(19) Europäisches Patentamt / European Patent Office / Office européen des brevets

(11) **EP 4 781 907 A1**

(12) **EUROPEAN PATENT APPLICATION**

(43) Date of publication:
**29.07.2026 Bulletin 2026/31**

(21) Application number: **26153755.9**

(22) Date of filing: **23.01.2026**

(51) International Patent Classification (IPC):
**A61B 5/08** (2006.01)  **A61B 5/00** (2006.01)

(52) Cooperative Patent Classification (CPC):
**A61B 5/082; A61B 5/7275; A61B 5/746;**
A61B 2503/22

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **24.01.2025 DE 102025000300**

(71) Applicant: **Airbus Defence and Space GmbH**
**82024 Taufkirchen (DE)**

(72) Inventors:
• **AUENHAMMER, Konrad**
**82024 Taufkirchen (DE)**
• **HORN, Stephan**
**82024 Taufkirchen (DE)**
• **KNAUP, Martin**
**82024 Taufkirchen (DE)**

(74) Representative: **LKGLOBAL**
**Partnerschaftsgesellschaft mbB**
**Brienner Straße 11**
**80333 München (DE)**

(54) **METHOD FOR CALCULATING A STRESS TOLERANCE RATING OF A FIGHTER PILOT**

(57) A new method uses a nitrogen monoxide (NO) sensor 10, which is arranged in an exhalation channel 3 of a breathing mask 1, which comprises a pressure ventilation device, an acceleration sensor 20, a ventilation pressure sensor 30, which is arranged in a breathing air channel 2 of the breathing mask 1, and comprises the following steps:

• a computer calculates an individual, unadjusted stress tolerance rating and uses for this purpose the measured value of the NO sensor 10 together with historical measured values and associated, historical calculation values,

• the computer calculates out the influence of the acceleration acting on the fighter pilot and the influence of the pressure ventilation acting on the fighter pilot and uses for this purpose measured values of the acceleration sensor 20 and of the ventilation pressure sensor 30 together with historical measured values of the acceleration sensor 20 and of the ventilation pressure sensor 30 and associated historical calculation values while obtaining an individualized, adjusted stress tolerance rating,

• wherein the historical measured values and the historical calculation values are stored in the memory and are data of the fighter pilot.

Fig.1

**Description**

The invention relates to a method for calculating an individualized, stress tolerance rating of a fighter pilot, wherein a biomarker sensor is used.

The term "measured value" is to be interpreted broadly and can comprise individual values, time-resolved values, integral values or differential values. Likewise, the term "calculation value" is to be interpreted broadly, since it relates to a measured value.

U.S. Pat. No. 8,892,274 B2 shows a method for calculating an individualized stress tolerance rating of a helicopter pilot for a helicopter assistance system. The method uses exclusively physiological sensors, such as, for example, a brain activity sensor, a respiratory rate sensor or a temperature sensor. The method further uses an evaluation device with a computer and a memory. The computer processes with a computer program the data of the sensors together with historical data of the helicopter pilot and calculates an individualized stress tolerance rating. The individualized stress tolerance rating controls an assistance system. A high stress tolerance rating requires a high level of support by the assistance system.

US 2021/0015399 A1 shows a biomarker sensor, with which the content of the biomarker nitrogen monoxide (NO) in combination with the biomarker isoprene can be detected. The biomarker sensor can be used to generate a hypoxia warning value for fighter pilots who are subjected to high accelerations.

In one of the numerous exemplary embodiments, U.S. Pat. No. 11,839,781 B1 shows, for example, a method for calculating an individualized hypoxia warning value of a fighter pilot. The method can be configured differently by selecting individual modules, as illustrated in FIG. 17 of U.S. Pat. No. 11,839,781 B1. In addition to many other sensors, an acceleration sensor and a ventilation pressure sensor, which is arranged in a breathing air channel of a breathing mask, can also be used. Although a wide variety of sensors are mentioned, an NO sensor, a biomarker sensor, is not mentioned. The method further uses a configurable evaluation device with a computer and a memory for storing historical data. The computer processes with a computer program data of the sensors together with historical data of the fighter pilot, in order, for example, to determine an individualized hypoxia warning value.

U.S. Pat. No. 12,085,507 B2 shows a sensor arrangement with a CO2 sensor and an H2O sensor for a method for calculating a hypoxia warning value. The sensor arrangement operates according to the principle of infrared spectroscopy. The sensor arrangement is arranged in an exhalation channel of the exhaled air of a breathing mask. A computer processes with a computer program the data of the sensors together with historical data of the fighter pilot and calculates a hypoxia warning value.

U.S. Pat. No. 10,595,758 B2 shows a hypoxia sensor which is a pulse oximeter.

The invention is based on the object of developing, starting from U.S. Pat. No. 8,892,274 B2, an alternative method for calculating a stress tolerance rating of a pilot. Further objects are the development of a corresponding computer program, a corresponding computer-readable medium and a corresponding device.

These objects are achieved according to the invention by claim 1 directed to a method, by claim 7 directed to a computer program, by claim 8 directed to a computer-readable medium and by claim 9 directed to a device.

The advantages of the invention are that a meaningful stress tolerance rating which is tailored exactly to fighter pilots is determined in a simple but reliable manner. A stress tolerance rating can be used to control an assistance system. If a stress tolerance rating falls, the assistance system has to take more tasks. The core idea of how a meaningful stress tolerance rating for fighter pilots is determined is that firstly a biomarker sensor, an NO sensor, is used which measures the content of NO. An unadjusted stress tolerance rating is derived therefrom. An adjustment takes place by a measured value being detected with an acceleration sensor, which measured value represents the influence of the acceleration acting on the fighter pilot, which acceleration represents a first environmental parameter to be factored out. A further adjustment takes place by the ventilation pressure sensor detecting a measured value, which represents the influence of the pressure ventilation acting on the fighter pilot, which pressure ventilation represents a second environmental parameter to be factored out. Historical data of the pilot are used for individualization.

Once again referring to the prior art presented above, U.S. Pat. No. 8,892,274 B2 does not comprise a biomarker in the method for calculating an individualized stress tolerance rating of a helicopter pilot for a helicopter assistance system. Another document, U.S. Pat. No. 2021/0015399 A1, admittedly proposes using an NO sensor, which is a biomarker sensor, in order to generate a hypoxia warning. However, no unadjusted stress tolerance rating is derived with the NO sensor, in which the influence of the acceleration acting on the fighter pilot and the influence of the pressure ventilation acting on the fighter pilot are factored out in order to obtain an individualized, adjusted stress tolerance rating.

An advantageous embodiment of the invention reproduces the features of claim 2. A simple formula is described which is comprised by the calculation steps of calculating an individualized, adjusted stress tolerance rating.

A further advantageous embodiment of the invention reproduces the features of claim 3. According to this, the NO sensor can be used as the sole biomarker sensor. Likewise, a second biomarker sensor can be used in combination with the NO sensor. The second biomarker is selected from a group which represents suitable biomarker sensors.

A further advantageous embodiment of the invention is shown by the features of claim 4. The temperature of the exhaled breath leads to the body temperature. The temperature sensor arranged in the exhalation channel 3 detects a measured value, which represents the influence of the body temperature of the fighter pilot, which influence is likewise to be factored out.

Further advantageous embodiments of the invention are shown by the self-explanatory features of claim 5 and of claim 6.

Exemplary embodiments of the invention are described in more detail with reference to the drawings. In the figures:

> FIG. 1 shows a breathing mask, sensors which are attached to the breathing mask, and an evaluation device, as a perspective schematic diagram,
> FIG. 2 shows a formula for calculating an individualized, adjusted stress tolerance rating of a fighter pilot.

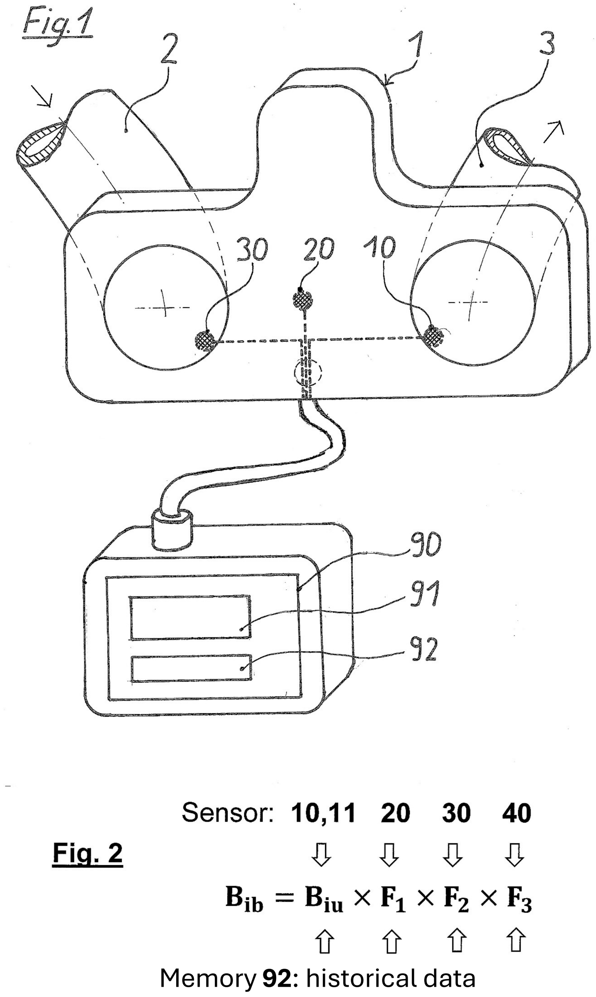

FIG. 1 illustrates a first exemplary embodiment, in which an individualized, adjusted stress tolerance rating $B_{ib}$ of a fighter pilot is calculated using a biomarker sensor and two environmental parameter sensors. The method uses, as the sole biomarker sensor, an NO sensor 10, which is arranged in an exhalation channel 3 of a breathing mask 1, which comprises a pressure ventilation device. For miniaturization, the NO sensor 10 comprises a ChemFET (chemical field-effect transistor). A ChemFET is a special type of field-effect transistor which is used for detecting chemical substances. It functions by converting changes in the chemical environment, such as the presence of certain molecules, into electrical signals.

The breathing mask 1 is attached to a helmet (not shown). The method further uses an acceleration sensor 20, which is arranged, for example, on the breathing mask 1 and records translational and rotational accelerations in all three spatial directions. The method uses a ventilation pressure sensor 30, which is arranged in a breathing air channel 2 of the breathing mask 1. The ventilation pressure sensor 30 operates non-intrusively according to the Doppler principle or according to the calorimetric principle. The acceleration sensor 20 and the ventilation pressure sensor 30 comprise MEMS (micro-electro-mechanical systems). MEMS are miniaturized systems which combine mechanical and electronic components on a single chip. Finally, the method uses an evaluation device 90 with a computer 91, a memory 92 and a real-time clock (RTC) device in order to provide the measured values of the sensors 10, 20, 30 with a time stamp.

The steps of the method are:

> a) the method uses an NO sensor 10, which is arranged in an exhalation channel 3 of a breathing mask 1, which comprises a pressure ventilation device, an acceleration sensor 20, a ventilation pressure sensor 30, which is arranged in a breathing air channel 2 of the breathing mask 1, a computer 91 and a memory 92, and comprises the following steps:
> b) the NO sensor 10, a biomarker sensor, detects a measured value of the NO content in the exhalation air of the fighter pilot,
> c) the acceleration sensor 20 detects a measured value, which represents the influence of the acceleration acting on the fighter pilot,
> d) the ventilation pressure sensor 30 detects a measured value, which represents the influence of the pressure ventilation acting on the fighter pilot, wherein it is noted that a breathing rate can also be determined, for example, with the ventilation pressure sensor 30,
> e) the computer calculates an individual, unadjusted stress tolerance rating $B_{iu}$ and uses for this purpose the measured value of the NO sensor 10 together with historical measured values and associated, historical calculation values,
> f) the computer calculates out the influence of the acceleration acting on the fighter pilot and the influence of the pressure ventilation acting on the fighter pilot and uses for this purpose measured values of the acceleration sensor 20 and of the ventilation pressure sensor 30 together with historical measured values of the acceleration sensor 20 and of the ventilation pressure sensor 30 and associated historical calculation values while obtaining an individualized, adjusted stress tolerance rating $B_{ib}$,
> g) wherein the historical measured values and the historical calculation values are stored in the memory and are data of the fighter pilot.

The individualized, adjusted stress tolerance rating $B_{ib}$ is calculated with calculation steps which comprise the following formula:

$$B_{ib} = B_{iu} \times F_1 \times F_2$$

wherein

- $B_{iu}$ is an individualized, unadjusted stress tolerance rating, calculated as the function value of the function $f(NO)$, wherein $f(NO)$ are historical calculation values of historical measured values of the NO sensor 10,
- $F_1$ is a first adjustment factor, calculated as the function value of the function $f(acceleration)$, wherein $f(acceleration)$ are historical calculation values of historical measured values of the acceleration sensor 20,
- $F_2$ is a second adjustment factor, calculated as the function value of the function $f(ventilation\ pressure)$, wherein $f(ventilation\ pressure)$ are historical calculation values of historical measured values of the ventilation pressure sensor 30.

The method operates autonomously and uses independent sensors 10, 11, 20, 30, 40, which are independent of avionics of an aircraft of the fighter pilot.

The individualized, adjusted stress tolerance rating $B_{ib}$ is used in an assistance system of a fighter aircraft of the fighter pilot and controls the degree of assistance.

The computer program comprises commands which cause the computer 91 to carry out the method steps. The computer program is stored on the internal memory of the computer 91 and as a backup on a computer-readable medium.

A second exemplary embodiment is described below with reference to a supplemented formula which is shown in FIG. 2. Only the differences with respect to the first exemplary embodiment are presented.

An unadjusted stress tolerance rating $B_{iu}$ is now calculated with an NO sensor 10 in combination with an isoprene sensor 11, a further biomarker sensor.

The method also uses a temperature sensor 40, which is arranged in the exhalation channel 3. The temperature sensor 40 detects a measured value, which represents the influence of the body temperature. This influence is factored out by the computer 91 using measured values of the temperature sensor 40 and associated historical calculation values. $F_3$ is a third adjustment factor, which is calculated as the function value of the function $f(temperature)$, wherein $f(temperature)$ are historical calculation values of historical measured values of the temperature sensor 40.

In contrast to the first and second exemplary embodiments, the following modifications are possible, for example:

   ▪ The second biomarker sensor in the second exemplary embodiment can also be an estradiol sensor, a 2-methylpentadecane sensor, an indole sensor, a benzaldehyde sensor, a 2-hydroxy-1-phenylethan-1-one sensor or a 2-ethylhexanol sensor.

   ▪ The method can additionally use a moisture sensor, which is arranged in the exhalation channel 3. The moisture sensor detects a measured value, which represents the influence of the dehydration acting on the fighter pilot. This influence is factored out by the computer using measured values of the moisture sensor and associated historical calculation values. A decreasing air humidity offers a conclusion about a possible dehydration associated with a mental performance drop.

   ▪ In the first and second exemplary embodiments, the method uses a ventilation pressure sensor 30, which is arranged in a breathing air channel 2 of the breathing mask 1. In addition, an exhalation pressure sensor, which is arranged in the exhalation channel 3, can be used. The breathing characteristic can be detected with an additional exhalation pressure sensor in order to more accurately determine the influence of the pressure ventilation on the fighter pilot.

Reference signs:

1    Breathing mask
2    Breathing air channel
3    Exhalation channel

10    NO sensor
11    Isoprene sensor
20    Acceleration sensor
30    Ventilation pressure sensor
40    Temperature sensor

90    Evaluation device
91    Computer
92    Memory

**Claims**

1. A method for calculating an individualized, adjusted stress tolerance rating ($B_{ib}$) of a fighter pilot, comprising the features:

   a) the method uses a nitrogen monoxide (NO) sensor (10), which is arranged in an exhalation channel (3) of a breathing mask (1), which comprises a pressure ventilation device, an acceleration sensor (20), a ventilation pressure sensor (30), which is arranged in a breathing air channel (2) of the breathing mask (1), a computer (91) and a memory (92), and comprises the following steps:
   b) the NO sensor (10), a biomarker sensor, detects a measured value of the NO content in the exhalation air of the fighter pilot,
   c) the acceleration sensor (20) detects a measured value, which represents the influence of the acceleration acting on the fighter pilot,
   d) the ventilation pressure sensor (30) detects a measured value, which represents the influence of the pressure ventilation acting on the fighter pilot,
   e) the computer calculates an individual, unadjusted stress tolerance rating ($B_{iu}$) and uses for this purpose the measured value of the NO sensor (10) together with historical measured values and associated, historical calculation values,
   f) the computer calculates the influence of the acceleration acting on the fighter pilot and the influence of the pressure ventilation acting on the fighter pilot and uses for this purpose measured values of the acceleration sensor (20) and of the ventilation pressure sensor (30) together with historical measured values of the acceleration sensor (20) and of the ventilation pressure sensor (30) and associated historical calculation values while obtaining an individualized, adjusted stress tolerance rating ($B_{ib}$),
g) wherein the historical measured values and the historical calculation values are stored in the memory (92) and are data of the fighter pilot.

2. Method according to claim 1, in which the individualized, adjusted stress tolerance rating ($B_{ib}$) is calculated with calculation steps which comprise the following formula:

$$B_{ib} = B_{iu} \times F_1 \times F_2$$

wherein

- $B_{iu}$ is an individualized, unadjusted stress tolerance rating, calculated as the function value of the function $f(NO)$, wherein $f(NO)$ are historical calculation values of historical measured values of the NO sensor (10),
- $F_1$ is a first adjustment factor, calculated as the function value of the function $f(acceleration)$, wherein $f(acceleration)$ are historical calculation values of historical measured values of the acce(leration sensor (20),
- $F_2$ is a second adjustment factor, calculated as the function value of the function $f(ventilation\ pressure)$, wherein $f(ventilation\ pressure)$ are historical calculation values of historical measured values of the ventilation pressure sensor (30).

3. Method according to claim 1 or 2, in which, for calculating an individualized, unadjusted stress tolerance rating ($B_{in}$), the NO sensor (10) is used either alone or in combination with a further biomarker sensor from a group consisting of an isoprene sensor (11), an estradiol sensor, a 2-methylpentadecane sensor, an indole sensor, a benzaldehyde sensor, a 2-hydroxy-1-phenylethan-1-one sensor, a 2-ethylhexanol sensor.

4. Method according to one of claims 1 to 3, in which the method uses a temperature sensor (40), which is arranged in the exhalation channel (3), wherein the temperature sensor (40) detects a measured value, which represents the influence of the body temperature of the fighter pilot and wherein this influence is factored out by the computer (91) using measured values of the temperature sensor (40) and associated historical calculation values.

5. Method according to one of claims 1 to 4, in which independent sensors (10, 11, 20, 30, 40), which are independent of avionics of an aircraft of the fighter pilot, are used.

6. Method according to one of claims 1 to 5, in which the individualized, adjusted stress tolerance rating ($B_{ib}$) is used in an assistance system of a fighter aircraft of the fighter pilot.

7. Computer program, comprising commands which cause the computer (91) of claim 1 to carry out the method steps according to claim 1.

8. Computer-readable medium, on which the computer program according to claim 7 is stored.

9. Device for calculating an individualized, adjusted stress tolerance rating ($B_{ib}$) of a fighter pilot, comprising the features:

a) the device comprises an NO sensor (10), which is arranged in an exhalation channel (3) of a breathing mask (1), which comprises a pressure ventilation device, an acceleration sensor (20), a ventilation pressure sensor (30), which is arranged in a breathing air channel (2) of the breathing mask (1), a computer (91) and a memory (92),
b) the device is designed to carry out the method according to one of claims 1 to 6.

Fig.1

Fig. 2

$$Sensor: \quad \mathbf{10,11} \quad \mathbf{20} \quad \mathbf{30} \quad \mathbf{40}$$

$$\Downarrow \qquad \Downarrow \qquad \Downarrow \qquad \Downarrow$$

$$\mathbf{B_{ib}} = \mathbf{B_{iu}} \times \mathbf{F_1} \times \mathbf{F_2} \times \mathbf{F_3}$$

$$\Uparrow \qquad \Uparrow \qquad \Uparrow \qquad \Uparrow$$

Memory **92**: historical data

Europäisches
Patentamt
European
Patent Office
Office européen
des brevets

**EUROPEAN SEARCH REPORT**

Application Number

EP 26 15 3755

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 2021/015399 A1 (GOUMA PELAGIA I [US]) 21 January 2021 (2021-01-21) <br> * abstract; figure 1 * <br> * paragraph [0009] - paragraph [0010] * <br> ----- | 1-9 | INV. <br> A61B5/08 <br> A61B5/00 |
| A | AU 2023 203 114 A1 (DRAEGER SAFETY AG & CO KGAA [DE]) 8 June 2023 (2023-06-08) <br> * abstract * <br> * paragraph [0053] * <br> * paragraph [0131] * <br> ----- | 1-9 | |
| A,D | US 11 839 781 B1 (DASHEVSKY DAVID [US] ET AL) 12 December 2023 (2023-12-12) <br> * abstract * <br> * column 8, line 49 - line 51 * <br> ----- | 1-9 | |

TECHNICAL FIELDS
SEARCHED     (IPC)

A61B

The present search report has been drawn up for all claims

| Place of search | Date of completion of the search | Examiner |
|---|---|---|
| The Hague | 20 May 2026 | Dhondt, Erik |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 4 781 907 A1**

## ANNEX TO THE EUROPEAN SEARCH REPORT
## ON EUROPEAN PATENT APPLICATION NO.

EP 26 15 3755

This annex lists the patent family members relating to the patent documents cited in the above-mentioned European search report.
The members are as contained in the European Patent Office EDP file on
The European Patent Office is in no way liable for these particulars which are merely given for the purpose of information.

20-05-2026

| Patent document cited in search report | Publication date | Patent family member(s) | Publication date |
|---|---|---|---|
| US 2021015399 A1 | 21-01-2021 | US 2021015399 A1 | 21-01-2021 |
| | | US 2024306935 A1 | 19-09-2024 |
| AU 2023203114 A1 | 08-06-2023 | AU 2021302337 A1 | 28-07-2022 |
| | | AU 2023203114 A1 | 08-06-2023 |
| | | BR 112022026734 A2 | 24-01-2023 |
| | | CA 3173467 A1 | 06-01-2022 |
| | | CN 115802936 A | 14-03-2023 |
| | | DE 102021111431 A1 | 30-12-2021 |
| | | EP 4171374 A1 | 03-05-2023 |
| | | EP 4736754 A2 | 06-05-2026 |
| | | EP 4736755 A2 | 06-05-2026 |
| | | JP 7634576 B2 | 21-02-2025 |
| | | JP 2023532017 A | 26-07-2023 |
| | | WO 2022002555 A1 | 06-01-2022 |
| US 11839781 B1 | 12-12-2023 | US 10561863 B1 | 18-02-2020 |
| | | US 11235183 B1 | 01-02-2022 |
| | | US 11524187 B1 | 13-12-2022 |
| | | US 11839781 B1 | 12-12-2023 |
| | | US 12303727 B1 | 20-05-2025 |
| | | US 12343575 B1 | 01-07-2025 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8892274 B2
- US 20210015399 A1
- US 11839781 B1
- US 12085507 B2
- US 10595758 B2